# EUROPEAN PATENT SPECIFICATION

(11) **EP 2 514 817 B1**
(45) Date of publication and mention of the grant of the patent: **30.08.2017**
(21) Application number: 12162270.8
(22) Date of filing: 29.03.2012
(51) Int. Cl.: C12N 5/0735

(54) **Non-tumorigenic expansion of pluripotent stem cells**
Nicht-tumorerzeugende Expansion pluripotenter Stammzellen
Expansion non oncogène de cellules souches pluripotentes

(30) Priority: 19.04.2011 US 201161476838 P
(43) Date of publication of application: 24.10.2012
(73) Proprietor: Hualien Tzu Chi Hospital, Buddhist Tzu Chi Medical Foundation, Hualien County 97002 (TW)
(72) Inventor: Ding, Dah-Ching, Hualien (TW); Chu, Tang-Yuan, Hualien (TW)
(74) Representative: Epping - Hermann - Fischer

(56) References cited:
- WO-A1-2009/148622
- CHO MEEYOUNG ET AL: "Human feeder layer system derived from umbilical cord stromal cells for human embryonic stem cells.", FERTILITY AND STERILITY 15 MAY 2010 LNKD- PUBMED:20403597, vol. 93, no. 8, 15 May 2010 (2010-05-15), pages 2525-2531, XP27047220, ISSN: 1556-5653
- AN SHI-MIN ET AL: "[Growing of human embryonic stem cells on feeders derived from themselves].", YI CHUAN = HEREDITAS / ZHONGGUO YI CHUAN XUE HUI BIAN JI DEC 2008 LNKD- PUBMED:19073571, vol. 30, no. 12, December 2008 (2008-12), pages 1567-1573, XP9161238, ISSN: 0253-9772
- HEMATTI PEIMAN: "Human embryonic stem cell-derived mesenchymal progenitors: an overview.", METHODS IN MOLECULAR BIOLOGY (CLIFTON, N.J.) 2011 LNKD- PUBMED:21042992, vol. 690, 1 April 2011 (2011-04-01), pages 163-174, XP9161182, ISSN: 1940-6029

### SEQUENCE LISTING

<110> Buddhist tzu chi general hospital
<120> Non-tumorigenic expansion of pluripotent stem cells
<130> 27134US
<160> 22
<170> PatentIn version 3.3
<210> 1
   <211> 26
   <212> DNA
   <213> artificial sequence
<220>
   <223> primer
<400> 1
   agtcccaaag gcaaacaacc cacttc 26
<210> 2
   <211> 27
   <212> DNA
   <213> artificial sequence
<220>
   <223> primer
<400> 2
   tgctggaggc tgaggtattt ctgtctc 27
<210> 3
   <211> 25
   <212> DNA
   <213> artificial sequence
<220>
   <223> primer
<400> 3
   cttgctgcag aagtgggtgg aggaa 25
<210> 4
   <211> 21
   <212> DNA
   <213> artificial sequence
<220>
   <223> primer
<400> 4
   ctgcagtgtg ggtttcgggc a 21
<210> 5
   <211> 18
   <212> DNA
   <213> artificial sequence
<220>
   <223> primer
<400> 5
   cccccggcgg caatagca 18
<210> 6
   <211> 20
   <212> DNA
   <213> artificial sequence
<220>
   <223> primer
<400> 6
   tcggcgccgg ggagatacat 20
<210> 7
   <211> 26
   <212> DNA
   <213> artificial sequence
<220>
   <223> primer
<400> 7
   gcgtcctggg aagggagatc cggagc 26
<210> 8
   <211> 26
   <212> DNA
   <213> artificial sequence
<220>
   <223> primer
<400> 8
   ttgaggggca tcgtcgcggg aggctg 26
<210> 9
   <211> 20
   <212> DNA
   <213> artificial sequence
<220>
   <223> primer
<400> 9
   tagtcagctg aagtgggaca 20
<210> 10
   <211> 20
   <212> DNA
   <213> artificial sequence
<220>
   <223> primer
<400> 10
   acgacaggtg cactttgtag 20
<210> 11
   <211> 20
   <212> DNA
   <213> artificial sequence
<220>
   <223> primer
<400> 11
   ctacaggggc acttaaccca 20
<210> 12
   <211> 20
   <212> DNA
   <213> artificial sequence
<220>
   <223> primer
<400> 12
   agagctgaat cgctcagagc 20
<210> 13
   <211> 20
   <212> DNA
   <213> artificial sequence
<220>
   <223> primer
<400> 13
   tgcctgagaa agagaaccag 20
<210> 14
   <211> 20
   <212> DNA
   <213> artificial sequence
<220>
   <223> primer
<400> 14
   atggcaggat gaacaaacac 20
<210> 15
   <211> 22
   <212> DNA
   <213> artificial sequence
<220>
   <223> primer
<400> 15
   gtcagctcaa tgccgacctc cg 22
<210> 16
   <211> 21
   <212> DNA
   <213> artificial sequence
<220>
   <223> primer
<400> 16
   gcagtggcgt cctggtactg c 21
<210> 17
   <211> 19
   <212> DNA
   <213> artificial sequence
<220>
   <223> primer
<400> 17
   ggcagcagca agcattcct 19
<210> 18
   <211> 18
   <212> DNA
   <213> artificial sequence
<220>
   <223> primer
<400> 18
   gcccaacacc cccagtca 18
<210> 19
   <211> 21
   <212> DNA
   <213> artificial sequence
<220>
   <223> primer
<400> 19
   agcctcatga agagccttcc a 21
<210> 20
   <211> 19
   <212> DNA
   <213> artificial sequence
<220>
   <223> primer
<400> 20
   tccggaagaa acccttgca 19
<210> 21
   <211> 18
   <212> DNA
   <213> artificial sequence
<220>
   <223> primer
<400> 21
   aggaggaggc agagcaca 18
<210> 22
   <211> 20
   <212> DNA
   <213> artificial sequence
<220>
   <223> primer
<400> 22
   ctggtatggc acaggtgatg 20

## Description

### BACKGROUND OF THE INVENTION

### 1. Field of the Invention

The present invention relates to expansion of human pluripotent stem cells, and particularly relates to non-tumorigenic expansion of human embryonic stem (hES) cells.

### 2. Description of Related Art

Human embryonic stem (hES) cells are pluripotent, and they have great ability to differentiate into almost all cell types of the adult. These cells therefore hold great promise for regenerative medicine. The special properties that make these hES cells desirable include immortality, pluripotency, and umlimited undifferentiated growth. Pluripotent embryonic stem cells are traditionally cultured on a layer of feeder cells such as mouse embryonic fibroblasts (MEFs) to keep them in an undifferentiated state. This feeder layer acts essentially to support the cells; and hES cell cultures are commonly cultured on a layer of the feeder layer until differentiation is desired. Unfortunately, these mouse feeder support cells are often associated with contamination to the hES cell cultures without much functional impact on them, thus rendering the hES cells that have been cultured on mouse feeder cells unsuitable for use clinically. It has been reported that human pluripotent stem (hPS) cells cultured without feeders soon die, or differentiate into a heterogeneous population of committed cells.

Numerous reports exist demonstrating that there have been attempts to replace the feeder or support cells using cell-free components, or at least to avoid non-human components or cells. The replacements have not shown long-term promising results, and such attempts have proven insufficient to support robust, continued propagation of cells. Furthermore, it has been shown that, in feeder-cell-free cultures, hES cells grown in medium replacements do form differentiated cells around the hES colonies, which is an indication that optimal conditions have not been achieved.

Therefore, a need exists for an alternative strategy for culturing hES cells by using another source of feeder cells that does not cause contamination and form teratomas.Meeyoung Cho et al. ("Human feeder layer system derived from umbilical cord stromal cells or human embryonic stem cells", Fertility and Sterility, 2010, 93, 2525-2531) discusses the potential of human umbilical cord-derived stromal cells (hUCSCs) as a human feeder for human embryonic stem cells (ESCs).

WO 2009/148622 A1 teaches methods for deriving and cultivating human embryonic stem cells.

An Shi-Min et al. ("Growing of human embryonic stem cells on feeders derived from themselves" Hereditas, 2008, 12, 30, 1567-1573) discloses mesenchymal stem cells derived from teratoma of human embryonic stem cells (hESCs) as feeder cells to support growth of hESCs.

Peiman Hematti et al. ("Human embryonic stem cell-derived mesenchymal progenitors: an overview" Methods in Molecular Biology 2011, 690, 163-174) describes mesenchymal stromal/stem cells and their use in the regeneration of human tissues.

KR 2011/0029591 describes a method for differentiating umbilical cord mesenchymal stem cells to nerve cells and hair cells to treat deafness and to develop novel drugs.

### SUMMARY OF THE INVENTION

The present invention refers to a method for non-tumorigenic expansion of human pluripotent stem cells, comprising co-culturing the human pluripotent stem cells with umbilical cord-derived stem cells in a medium, wherein the umbilical cord-derived stem cells are human umbilical cord-derived mesenchymal stem cells (HUCMSCs) and the medium comprises 2 mM L-glutamine.

The umbilical cord-derived stem cells form a feeder layer in a medium for the expansion of the human pluripotent stem cells, and maintain the human pluripotent stem cells in an undifferentiated state.

In one embodiment, the expansion of the human pluripotent stem cells is non-tumorigenic expansion, such that the human pluripotent stem cells are free from forming teratoma.

In another embodiment, the umbilical cord-derived stem cells are positive for CD10, CD13, CD29, CD44, CD73, CD90, CD166 or HLA-ABC, but negative for CD1q, CD3, CD34, CD45, CD56, CD117 or HLA-DR. Also, the umbilical cord-derived stem cells have osteogenic or adipogenic differentiability.

In another embodiment, the umbilical cord-derived stem cells are human umbilical cord-derived mesenchymal stem cells (HUCMSCs), and are derived from Wharton's jelly of a human umbilical cord.

In another embodiment, the human pluripotent stem cells are positive for alkaline phosphatase (AP), Oct-4, SSEA-1, SSEA-4, TRA-1-60, TRA-1-81, NANOG, SOX2, NF-200, brachyury, ATBF1 or MAP2, and express *GDF9, GATA4, HAND1* or *TUJ-1* genes. Also, MYC is down-regulated in the human pluripotent stem cells.

In another embodiment, the human pluripotent stem cells are human embryonic stem (hES) cells, and form embryiod bodies.

In addition, a medium for expansion of human pluripotent stem cells is described, wherein the medium includes umbilical cord-derived stem cells that form a feeder layer in the medium. The umbilical cord-derived stem cells in the medium can be human umbilical cord-derived mesenchymal stem cells (HUCMSCs).

Furthermore, a kit for expansion of human embryonic stem (hES) cells is described, wherein the kit comprises a medium including umbilical cord-derived stem cells, and instructions for the use thereof. The umbilical cord-derived stem cells in the medium of the kit can be human umbilical cord-derived mesenchymal stem cells (HUCMSCs).

### BRIEF DESCRIPTION OF THE DRAWINGS

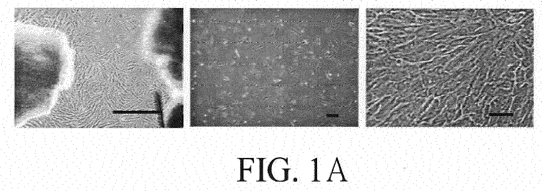

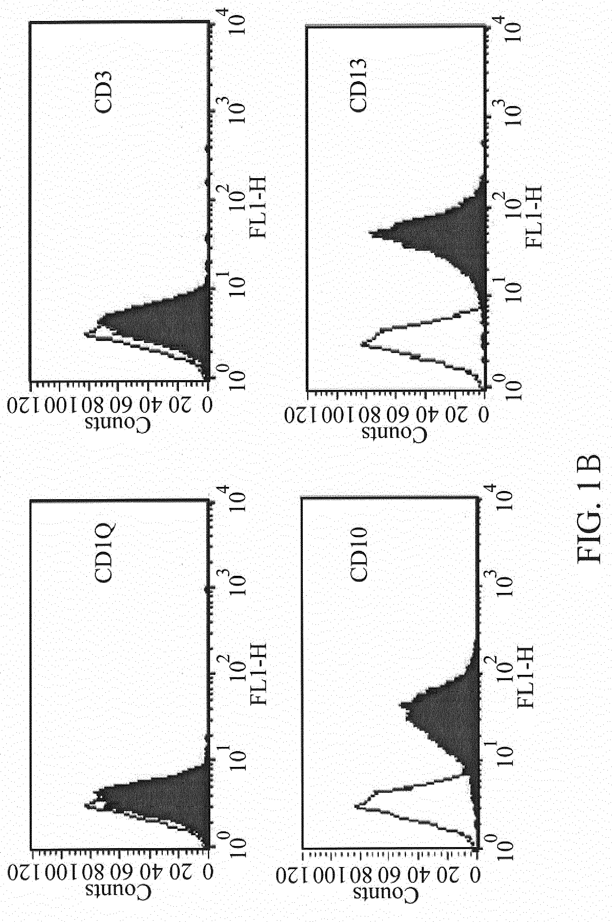

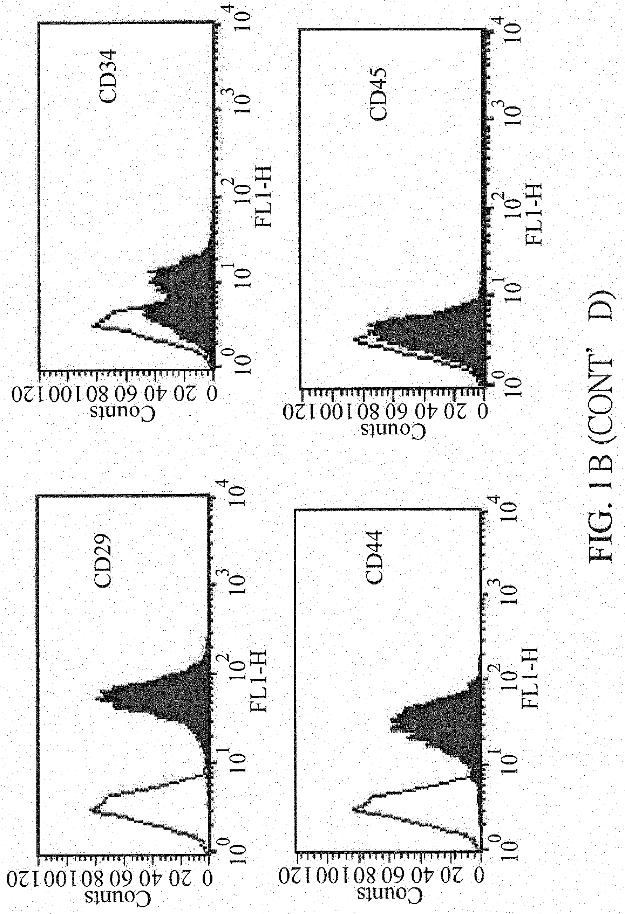

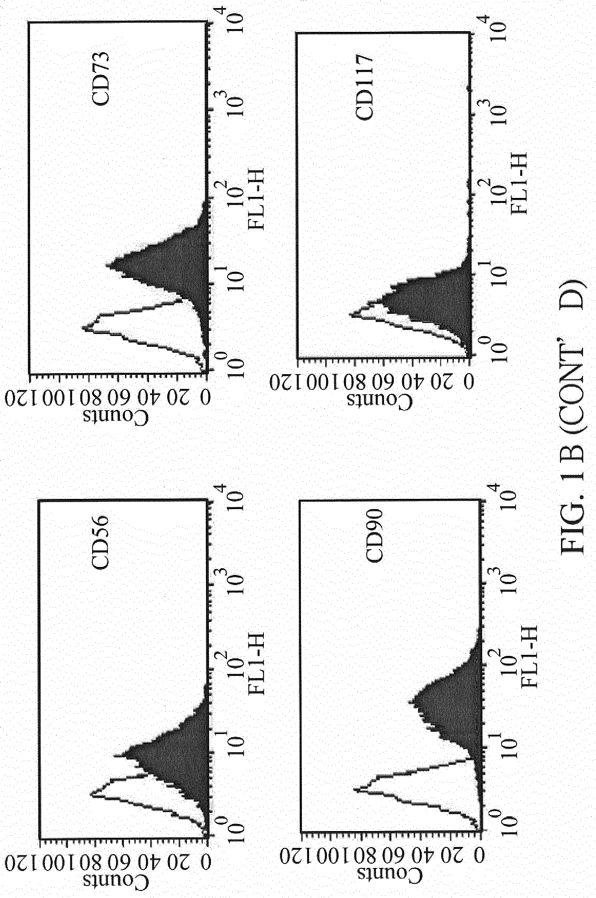

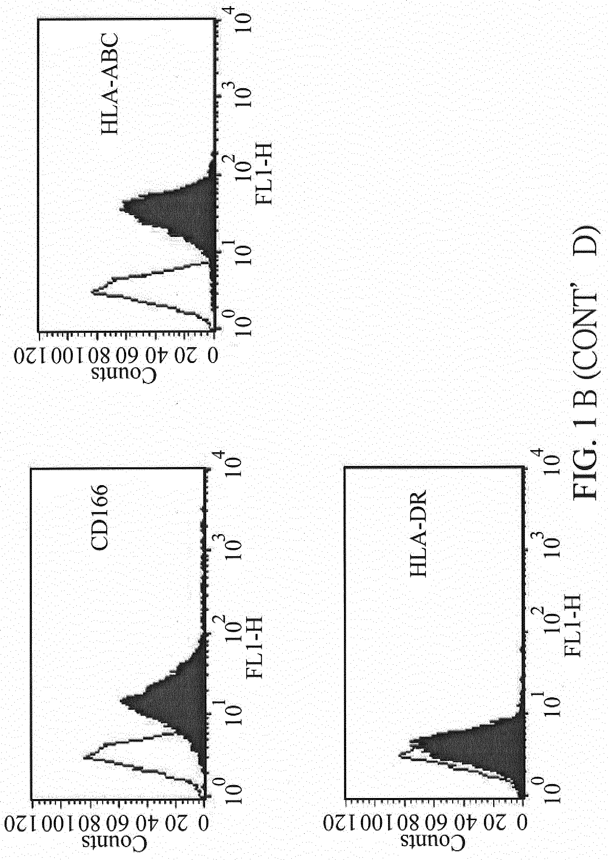

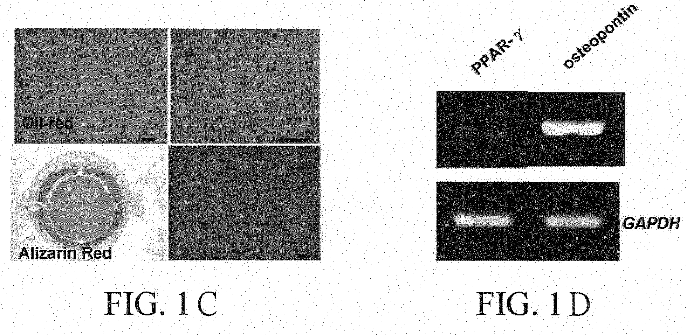

Figure 1 shows morphology, immunotyping and *in vitro* differentiation of HUCMSC. Cells of Wharton's jelly (WJ) growing from explants are fibroblast-like with a spindle-shaped morphology (Figure 1A). Flow cytometry of rapidly dividing HUCMSCs showed negative for CD1q, CD3, CD34, CD45, CD56, CD117 and HLA-DR, and positive for CD10, CD13, CD29, CD44, CD73, CD90, CD166 and HLA-ABC (Figure 1B). Upon adipogenic differentiation, the cells formed neutral lipid vacuoles and contained numerous Oil-Red-O positive lipid droplets (Figure 1C, the upper panel). In osteogenic medium, the cells broadened to form a mineralized matrix, which was strongly stained with Alizarin Red S (Figure 1C, the lower panel) after three to four weeks of cultivation. Expression of genes specific for adipogenic (PPARγ) and osteogenic (osteopontin) differentiation was showed by RT-PCR analysis with GAPDH as a positive control (Figure 1D). Scale bars represent 1000 µm in the left panel of Figure 1A and 100 µm in the right two panels of Figure 1A and in Figure 1C.

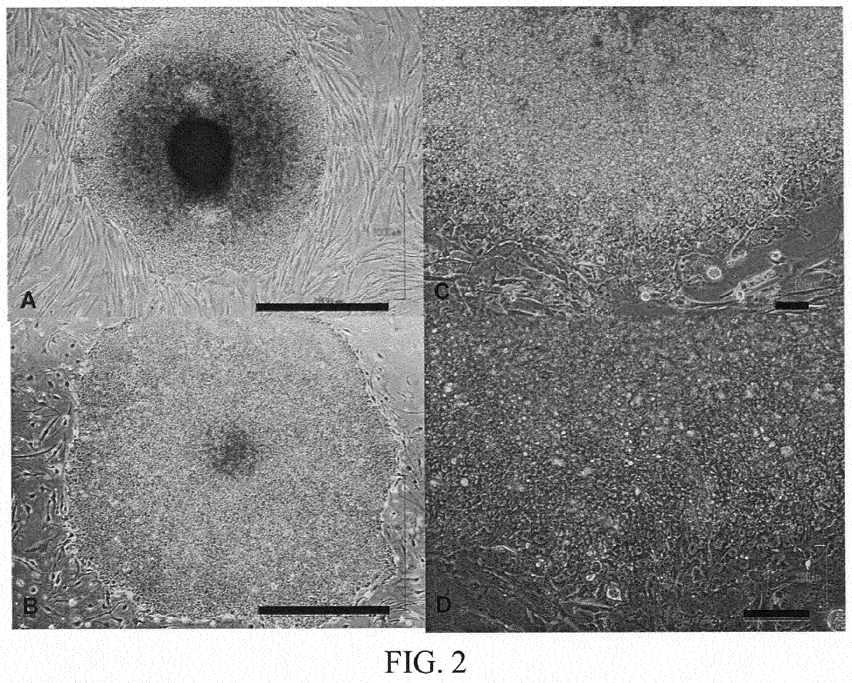

Figure 2 shows morphology of undifferentiated human ES cell colonies grown on HUCMSC and MEF feeders. Pictures of the hES colony grown on HUCMSC (Figure 2A) and MEF (Figure 2B) feeder layers are shown. A magnification of colony grown on HUCMSC revealed typical hES cell morphology with high nucleus: cytoplasm ratio (Figures 2C and 2D). Scale bars represent 1000 µm in Figures 2A and 2B and 100 µm in Figures 2C and 2D.

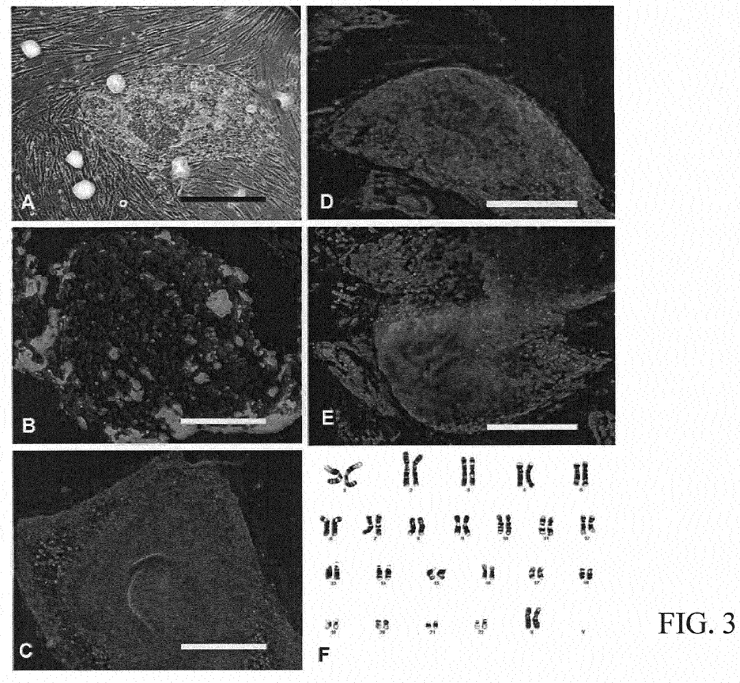

Figure 3 shows phenotypes of human ES cells cultured on HUCMSC. Immunostaining of the hES colony with specific antibodies revealed strong expression of alkaline phosphatase (Figure 3A), Oct4 (Figure 3B), SSEA-4 (Figure 3C), TRA-1-60 (Figure 3D), and TRA-1-81 (Figure 3E). A representative normal karyotype (46, XX) was observed in ES cells after 20 passages of continuous culture on HUCMSC (Figure 3F). Scale bars represent 1000 µm.

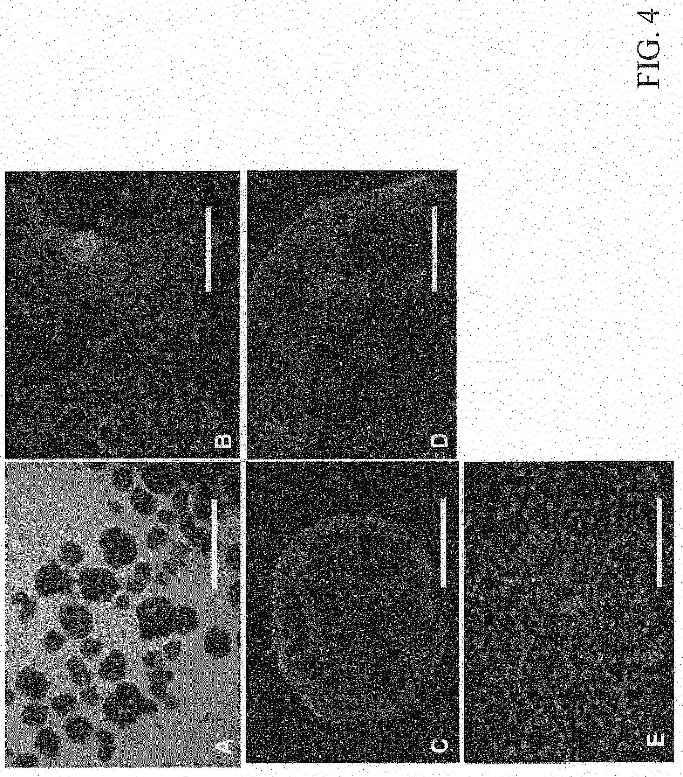

Figure 4 shows fluorescence immunostaining of embryonic bodies (EB) derived from hES. Embryonic bodies are shown under phase contrast microscope (Figure 4A), and by immunostaining with antibodies against NF-200 (Figure 4B), brachyury (Figure 4C), ATBF1 (Figure 4D), and MAP2 (Figure 4E). Scale bars represent 1000 µm.

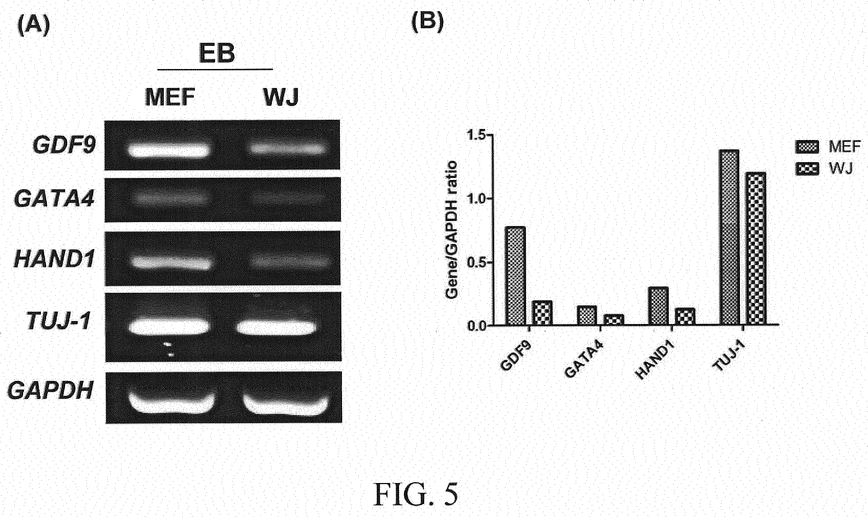

Figure 5 shows expression of differentiation markers in hES cells on different feeders. Figure 5A illustrates expression of genes that are specific for germ cell *(GDF9),* endoderm (*GATA4*), mesoderm (*HAND1*) and ectoderm (*TUJ-1*) by means of RT-PCR. *GAPDH* was used as a control. Figure 5B illustrates semi-quantitative analysis of gene expression shown in Figure 5A.

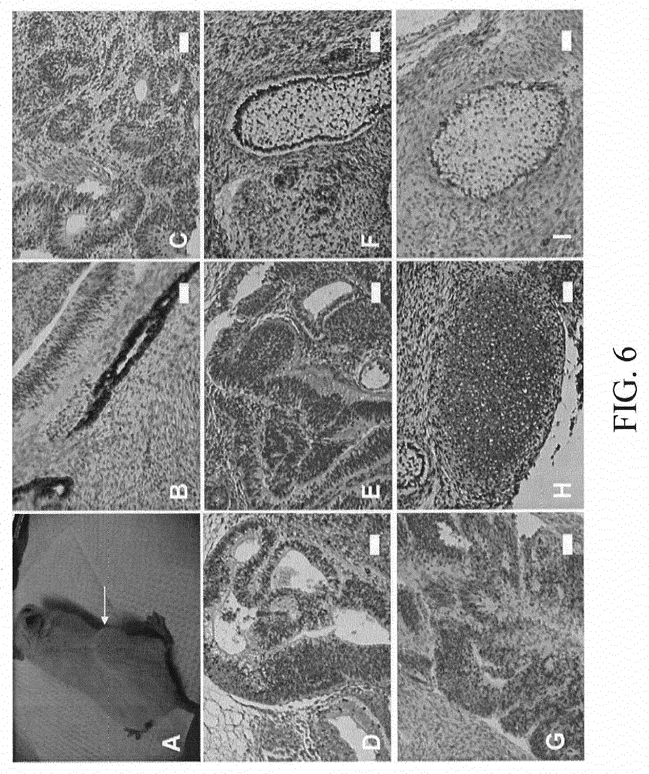

Figure 6 shows teratomas developed from hES cells with a change of the feeder cells from HUCMSC to MEF. Figure 6A shows teratoma (indicated by the arrow) readily developed from hES cells after change of the feeder from HUCMSC to MEF on NOD-SCID mice. Histological section revealed ribbon of melanocytes with a retina-like structure (Figure 6B), neurotube-like structures (Figures 6C-6E), odontogenic epithelium (Figure 6F), neuroepithelium (Figure 6G), immature cartilage (Figure 6H) and immature squamoid epithelium (Figure 6I). Scale bars represent 100 µm.

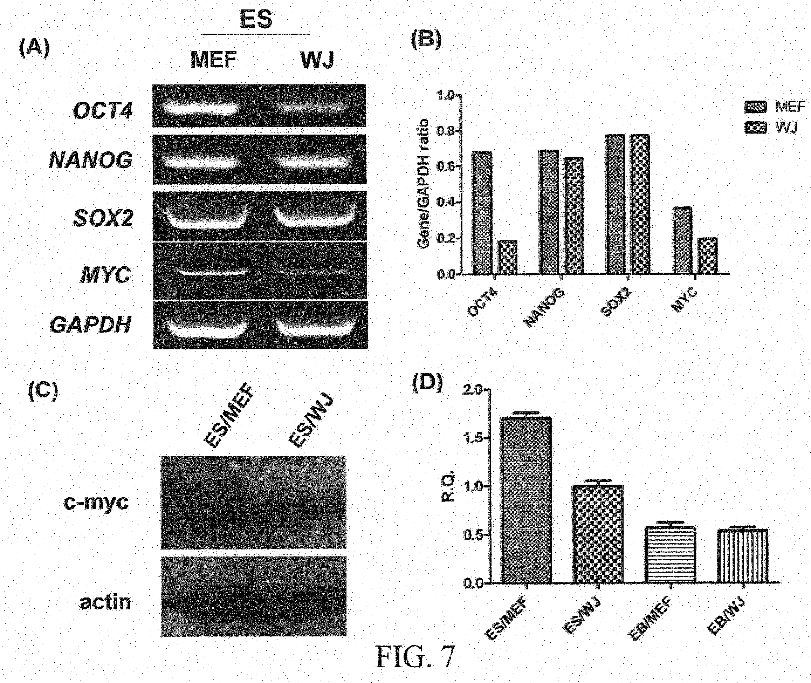

Figure 7 shows expression of pluripotent genes in hES cells cultured on HUCMSC and MEF. Figure 7A illustrates RT-PCR analysis of *OCT4, NANOG, SOX2 and MYC* in hES cells (ES) cultured on HUCMSC (WJ) and MEF with GAPDH as the internal control. Figure 7B illustrates semi-quantitative analysis of gene expression shown in Figure 7A. Figure 7C illustrates the C-MYC protein measured by Western blot analysis. Figure 7D illustrates the mRNA levels of *MYC* measured by qRT-PCR as revealed by folds of the internal control. EB stands for the embryoid body.

### DETAILED DESCRIPTION OF THE PREFERRED EMBODIMENTS

Various specific details are provided herein to provide a more thorough understanding of the invention.

### RT-PCR and quantitative RT-PCR of the gene and other differentiation marker genes

Undifferentiated or differentiated hES cells that had been cultured on HUCMSC or MEF feeder layers were removed mechanically and treated with RLT lysis buffer (Qiagen). The first strand of cDNA was synthesized using a SuperScript III One-Step RT-PCR kit (Invitrogen) following the manufacturer's instructions. Table 1 presents the sequence, annealing temperature and product size of each pair of primers. All PCR samples were analyzed by electrophoresis on 2% agarose gel that contained 0.5 µg/ml ethidium bromide (Sigma). For quantitative RT-PCR (qRT-PCR) analysis, FastStart universal SYBR green master (ROX, Roche, USA) gene expression assays was used in an ABI Step One Plus system (Applied Biosystems), with *GAPDH* used as an internal control. The sequences of primers and annealing temperatures are shown in Table 1.

**Table 1**

| Gene | Sense (5'-3') | Antisense (5'-3') | Annealing Temp (°C) | Product size (bp) |
|---|---|---|---|---|
| *Nanog* | AGTCCCAAAGGCAAACAACCCACTTC | TGCTGGAGGCTGAGGTATTTCTGTCTC | 55 | 164 |
| *Oct3*/*4* | CTTGCTGCAGAAGTGGGTGGAGGAA | CTGCAGTGTGGGTTTCGGGCA | 55 | 171 |
| *SOX2* | CCCCCGGCGGCAATAGCA | TCGGCGCCGGGGAGATACAT | 55 | 448 |
| *MYC* | GCGTCCTGGGAAGGGAGATCCGGAGC | TTGAGGGGCATCGTCGCGGGAGGCTG | 55 | 328 |
| *GDF9* | TAGTCAGCTGAAGTGGGACA | ACGACAGGTGCACTTTGTAG | 55 | 278 |
| *GATA4* | CTACAGGGGCACTTAACCCA | AGAGCTGAATCGCTCAGAGC | 60 | 157 |
| *Hand1* | TGCCTGAGAAAGAGAACCAG | ATGGCAGGATGAACAAACAC | 55 | 273 |
| *Tuj-1* | GTCAGCTCAATGCCGACCTCCG | GCAGTGGCGTCCTGGTACTGC | 55 | 559 |
| *GAPDH* | GGCAGCAGCAAGCATTCCT | GCCCAACACCCCCAGTCA | 60 | 136 |
| *PPAR-r* | AGCCTCATGAAGAGCCTTCCA | TCCGGAAGAAACCCTTGCA | 60 | 120 |
| *osteopontin* | AGGAGGAGGCAGAGCACA | CTGGTATGGCACAGGTGATG | 60 | 150 |

### Isolation and expansion of HUCMSCs

Human umbilical cord samples (20 cm in length, 20 g in weight) were collected in sterile boxes that contained Hanks' balanced salt solution (HBSS; Gibco/BRL 14185-052). The protocols for collecting and using human umbilical cord were approved by the Institutional Review Board of Tzu-Chi University Hospital. Written informed consent was obtained from pregnant women before labor pain.

Collected human umbilical cord tissues were washed three times in Ca²⁺ and Mg²⁺-free phosphate buffered saline (Dulbecco's PBS, Life Technology). They were mechanically cut along the midline, and the umbilical arteries, vein and outlining amniotic membrane were dissociated from the WJ. The jelly was then cut into pieces that were smaller than 0.5 cm³, treated with trypsin/EDTA (Sigma, St. Louis, USA), and incubated for 30 min at 37°C in a 95% air / 5% CO₂ humidified atmosphere. The explants were then cultured in Dulbecco's Modified Eagle Medium (DMEM) that contained 10% human umbilical cord blood serum (CBS) and antibiotics and left undisturbed for five to seven days to allow the cells to migrate from the explants.

### Characteristics of HUCMSCs derived from human umbilical cord

The MSC specific surface markers were characterized by flow cytometric analysis. The cells were detached using 2 mM EDTA in phosphate buffered saline (PBS), washed and incubated with the appropriate antibody that was conjugated with fluorescein isothiocyanate (FITC) or phycoerythrin (PE). The antibodies were CD1q, CD3, CD10, CD13, CD14, CD29, CD31, CD34, CD44, CD45, CD49b, CD49d, CD56, CD73, CD90, CD105, CD117, CD166, HLA-ABC and HLA-DR (BD, PharMingen). Then, the cells were analyzed using a Becton Dickinson flow cytometer (Vantage SE, Becton Dickinson, San Jose, CA).

Upon the first culturing of WJ tissue pieces, attached growing cells with a spindle-shaped morphology migrated from the explants (FIG 1A). These cells divided rapidly with a doubling time of 28 h, and underwent more than 25 passages (equivalent to over 40 population doublings), without spontaneous differentiation. They were negative for CD1q, CD3, CD34, CD45, CD56, CD117, and HLA-DR, and positive for CD10, CD13, CD29, CD44, CD73, CD90, CD166 and HLA-ABC (FIG. 1B). These observations demonstrate that cells that are isolated from WJ of the human umbilical cord have the same surface markers as mesenchymal stem cells (MSCs).

### In vitro differentiation of HUCMSCs to osteocytes and adipocytes

To induce osteogenic and adipogenic differentiations, HUCMSCs were transferred to an osteogenic medium (DMEM supplemented with 10% CBS, 0.1 µmol/L dexamethasone, 10 mmol/L β-glycerol phosphate, 50 µmol/L ascorbate) and adipogenic medium (DMEM supplemented with 10% CBS, 1 µmol/L dexamethasone, 5 µg/mL insulin, 0.5 mmol/L isobutylmethylxanthine and 60 µmol/L indomethacin) for three weeks. The potential for osteogenesis was evaluated by determining the mineralization of calcium by staining with Alizarin Red S (Sigma, USA). To assess adipogenic differentiation, intracellular lipid droplets were observed under a microscope and the lipid droplet was verified by staining with Oil Red O.

Adipogenic differentiation of HUCMSC was apparent two weeks after incubation with an adipogenic medium supplement. At the end of the second week, changes were evident in the cell morphology, and in the formation of neutral lipid vacuoles: almost all cells contained many Oil Red-O-positive lipid droplets (FIG. 1C). Similarly, the induction of differentiation in the osteogenic medium caused the treated cells to grow rapidly and contain mineralized matrices, which were strongly stained by Alizarin Red-S, indicating deposition of calcium after three to four weeks of cultivation (FIG. 1C). Expression of adipogenic (*PPARγ*) and osteogenic (*Osteopontin*) genes was evident in RT-PCR analysis (FIG. 1D).

### Characterization of HUCMSC-co-cultured reference hES cell lines

The reference TW1 cell line (P22, i.e., the twenty-second passage) was obtained from Biomedical Technology and Device Research Laboratories, Industrial Technology Research Institute, and initially cultured on MEFs, as directed by the supplier, i.e., the Food Industry Research and Development Institute, FIRDI, Taiwan. Either MEFs (P3, the third passage) or HUCMSCs after mitomycin-C deactivation were used as feeder cells for culturing the hES cells (reference cell line). They were plated at a density of 200,000 cells per 9.4 cm² per well in six-well plates. The hES cell culture medium comprised 80% (v/v) knockout (KO) DMEM, 20% (v/v) KO serum replacement, 2 mM L-glutamine, 10 mM nonessential amino acids (all from Invitrogen), 50 µM B-mercaptoethanol (Sigma), and 4 ng/mL bFGF.

The hES cells were characterized by immunocytochemistry using fluorescence-labeled antibodies specific for undifferentiated hES cells, which were SSEA-4, SSEA-1, TRA-1-60, TRA-1-81 and Oct-4 (ES Cell Characterization Kit; Chemicon). Initially, hES cells were cultured on chamber slides (Nunc, Denmark) or sterile cover glasses (Assistent, Germany) in culture dishes with feeder cells. At specified times on days three to seven following passage, the colonies of the hES cells were subjected to immunofluorescence staining. Briefly, cells were fixed with 4% paraformaldehyde and then underwent several procedures, including permeabilization (0.1% Triton X-100), blocking (4% normal goat serum), treatment with primary antibody (1:10 - 1:50 dilution), three washings, treatment with fluorescent-labeled secondary antibody, three more washings, covering with a coverslip and mounting. Alkaline phosphatase (AP) staining was conducted using the ES Cell Characterization Kit (Chemicon).

The hES cells that were transferred to the HUCMSCs feeders formed colonies effectively and continued to proliferate with a doubling time of 36 hours. The morphology of colonies differed slightly from that of those that were cultured on MEF (FIGs. 2A and 2B). However, the individual human ES cell morphology cultured on HUCMSC remained the same as on MEF. The cells remained round and small, with a high nucleus: cytoplasm ratio, and the notable presence of one to three nucleoli (FIGs. 2C and 2D). The TW1 hES cells on HUCMSCs feeders expressed AP, Oct-4, SSEA-4, TRA-1-60 and TRA-1-81 markers (FIGs. 3A-3E).

The karyotypes of cells were studied at passage 40. On day seven after passage, hES cells were treated with 0.1 µg/ml colcemid (Gibco) for 4 h. After the cells were washed, they were treated with either 0.25% trypsin for 3-5 min or collagenase type IV for 8 min, pipetted and harvested. They were then fixed and mounted on glass slides. The metaphases were analyzed using the standard G-banding method in a certified cytogenetic laboratory. At passage 40, the TW1 hES cells on HUCMSCs feeders revealed normal karyotypes of 46, XX (FIG. 3F).

### In vitro pluripotency of HUCMSC-co-cultured reference hES cell line

Before differentiation, hES cells were collected and resuspended in the hES cell medium in the absence of bFGF. The hES cells were then cultured in low attachment six-well plates as aggregates in suspension. After five days, fetal bovine serum (FBS) (5% final) was added. Aggregated hES cells usually formed an embryoid body (EB) after seven to ten days, and mature (cystic) EBs subsequently emerged from 20% to 80% of the formed EBs. The resultant solid or cystic EB was then plated on gelatin-treated chamber slides or 35 mm culture dishes for further differentiation and was then subsequently treated similarly to those cells that were grown directly in an adherent culture. After fixation, the differentiated hES cells were studied by immunocytochemistry using fluorescence-labeled antibodies specific for three embryonic germ layers, which were MAP2, NF200 (Chemicon) for ectoderm, brachyury for mesoderm and ATBF1 (Santa Cruz) for endoderm.

Like those grown on MEFs, hES cells cultured with HLTCMSC feeders formed EBs when cultured in suspension. Within these EBs, cell differentiation that represented three embryonic germ layers was observed. These cells expressed ectodermal markers (MAP-2 and NF-200), mesodermal marker (brachyury) and endodermal marker (ATBF1), as indicated by immunohistochemistry (FIG. 4). Cells that were harvested from seven to ten day-old EBs expressed such genes as GDF9 (germ cell related), *GATA4* (endoderm), *Hand1* (mesoderm) and Tuj-1 (ectoderm), as indicated by RT-PCR (FIG. 5).

### Non-tumorigenesis and in vivo pluripotency of HUCMSC-co-cultured reference cell line

hES cells were detached with mechanical slicing using glass capillaries, pelleted, resuspended in PBS with Matrigel (BD Biosciences) (1:1) and injected into the back subcutaneous tissue (n=17) or renal capsule (n=4) of non-obese diabetic-severe combined immunodeficiency (NOD-SCID) mice. Cells were counted using a hemocytometer and suspended in PBS with Matrigel (1:1) in various concentrations. hES cells were kept on ice less than 45 min for optimal viability prior to injection. The teratoma formation was followed up by palpation and resulting tumors were dissected, fixed, embedded in paraffin and processed for histology.

The developmental potential of cells after long-term culture on HUCMSC feeders was investigated *in vivo* using a xenograft model. In extensive trials with 21 different transplantations in both NOD-SCID (n=18) and nude (n=3) mice, no teratoma growth was observed during long-term followed up for over three months (Tables 2 and 3). However, teratomas were clearly observed upon transient (for days) subsequent culturing on MEF feeders. The histologies of the teratomas that were derived from the hES cells before and after they were transferred to the HUCMSC feeder did not differ (Table 2 and FIG. 6).

**Table 2. Summary of xenograft experiments with TW1 human embryonic stem cells alternatively cultured on MEF and HUCMSC as feeders**

| Xenograft date | Feeder layer | Teratoma / injection numbers |
|---|---|---|
| Jan. 2008 | MEF | 2/2 |
| April 2008 - June 2009 | HUCMSC | 0/21 |
| Mar. 2009 | MEF | 2/2 |

**Table 3. Trials of xenograft tumorigenesis of human embryonic stem cells co-cultured with HUCMSCs**

| Xenograft date | Injected cell numbers | Mouse | Mouse number | Site of injection |
|---|---|---|---|---|
| Apr. 18,2008 | 2 x 10⁵ | NOD-SCID | 2 | s.c.* |
| Jun. 9, 2008 | 3 x 10⁵ | NOD-SCID | 2 | s.c. |
| Jul. 8, 2008 | 5 x 10⁵ | NOD-SCID | 2 | s.c. |
| Jul. 16, 2008 | 2 x 10⁶ | NOD-SCID | 2 | s.c. |
| Jan. 8,2009 | 1.5 x 10⁶ | nude mice | 2 | s.c. |
| Jan. 22,2009 | 3 x 10⁵ | nude mice | 1 | s.c. |
| Mar. 6,2009 | 1 x 10⁶ | NOD-SCID | 2 | s.s |
| Mar. 19,2009 | 5 x 10⁶ | NOD-SCID | 3 | s.c. |
| Apr. 30,2009 | 3 x 10⁷ | NOD-SCID | 2 | s.c. |
| Jun. 19,2009 | 7 x 10⁵ | NOD-SCID | 4 | renal capsule |

| | | | | |
|---|---|---|---|---|
| * s.c.: subcutaneous | | | | |

### Down-regulation of MYC in reference hES co-cultured with HUCMSC

Cells were lysed in the lysis buffer (150 mM NaCl, 50 mM Tris-HCl [pH 7.4], 1% Nonidet P-40) plus proteinase inhibitor cocktail (Roche, Indianapolis, IN). Proteins were electrophoresed on 10% sodium dodecyl sulfate-polyacrylamide gel electrophoresis (SDS-PAGE) and then transferred to a nitrocellulose membrane (Hybond-C Super; Amersham, Little Chalfont, UK). The membranes were incubated with anti-c-myc (2 µg/m) or anti-α-actin (1:10,000; Sigma-Aldrich) monoclonal antibodies. HRP-conjugated goat anti-mouse IgG (Jackson Immuno-Research Laboratories) was used as the secondary antibody. Bound antibodies were detected using enhanced chemiluminescence reagents (ECL; Amersham).
The expression of key pluripotency genes in hES on the two different feeders was further tested. Markers of undifferentiated stem cells, such as *Oct-4, Nanog,* and *Sox2* were readily expressed as indicated by RT-PCR (FIGs. 7A and 7B). Lower expressions of the homeobox gene OCT4 and proto-oncogene MYC were observed in hES co-cultured with HUCMSC (FIGs. 7A and 7B). This down-regulation of MYC was further proved by Western blot analyses and quantitative RT-PCR (FIGs. 7C and 7D).

## Claims

1. A method for non-tumorigenic expansion of human pluripotent stem cells, comprising co-culturing the human pluripotent stem cells with umbilical cord-derived stem cells in a medium, wherein the umbilical cord-derived stem cells are human umbilical cord-derived mesenchymal stem cells (HUCMSCs) and the medium comprises 2 mM L-glutamine.

2. The method of claim 1, wherein the human umbilical cord-derived mesenchymal stem cells form a feeder layer in a medium for the expansion of the human pluripotent stem cells.

3. The method of claim 1 or 2, wherein the human umbilical cord-derived mesenchymal stem cells maintain the human pluripotent stem cells in an undifferentiated state.

4. The method of one of claims 1-3, wherein the human umbilical cord-derived mesenchymal stem cells are positive for one or more of CD10, CD13, CD29, CD44, CD73, CD90, CD166 and HLA-ABC, and/or wherein the human umbilical cord-derived mesenchymal stem cells are negative for one or more of CD1q, CD3, CD34, CD45, CD56, CD117 and HLA-DR.

5. The method of any of the preceding claims, wherein the human umbilical cord-derived mesenchymal stem cells have osteogenic or adipogenic differentiability.

6. The method of any of the preceding claims, wherein the human pluripotent stem cells are human embryonic stem (hES) cells.

7. The method of any of the preceding claims, wherein the human pluripotent stem cells are positive for one or more of alkaline phosphatase (AP), Oct-4, SSEA-1, SSEA-4, TRA-1-60, TRA-1-81, NANOG, SOX2, and a differentiation marker, and wherein the differentiation marker is one or more of NF-200, brachyury, ATBF1 and MAP2.

8. The method of any of the preceding claims, wherein the human pluripotent stem cells express one or more of differentiation genes, and wherein the differentiation genes are *GDF9, GATA4, HAND1* or *TUJ-1* genes.

9. The method of any of the preceding claims, wherein MYC is down-regulated in the human pluripotent stem cells.

10. The method of any of the preceding claims, wherein the human pluripotent stem cells form embryoid bodies.

## Patentansprüche

1. Verfahren zur nicht-tumorerzeugenden Expansion humaner pluripotenter Stammzellen, umfassend das Co-Kultivieren der humanen pluripotenten Stammzellen mit aus der Nabelschnur stammenden Stammzellen in einem Medium, wobei die aus der Nabelschnur stammenden Stammzellen aus der Nabelschnur stammende mesenchymale Stammzellen (HUCMSCs) sind und das Medium 2 mM L-Glutamin aufweist.

2. Verfahren nach Anspruch 1, wobei die aus der Nabelschnur stammenden mesenchymalen Stammzellen eine Feeder-Schicht in einem Medium zur Expansion der humanen pluripotenten Stammzellen bilden.

3. Verfahren nach Anspruch 1 oder 2, wobei die aus der Nabelschnur stammenden mesenchymalen Stammzellen die humanen pluripotenten Stammzellen in einem undifferenzierten Zustand halten.

4. Verfahren nach einem der Ansprüche 1-3, wobei die aus der Nabelschnur stammenden mesenchymalen Stammzellen positiv für eines oder mehr aus CD10, CD 13, CD29, CD44, CD73, CD90, CD166 und HLA-ABC sind, und/oder wobei die aus der Nabelschnur stammenden mesenchymalen Stammzellen negativ für eines oder mehr aus CD1q, CD3, CD34, CD45, CD56, CD 117 und HLA-DR sind.

5. Verfahren nach einem der vorangehenden Ansprüche, wobei die aus der Nabelschnur stammenden mesenchymalen Stammzellen eine osteogene oder adipogene Differenzierbarkeit haben.

6. Verfahren nach einem der vorangehenden Ansprüche, wobei die humanen pluripotenten Stammzellen humane embryonale Stammzellen (hES) sind.

7. Verfahren nach einem der vorangehenden Ansprüche, wobei die humanen pluripotenten Stammzellen positiv für eines oder mehr aus alkalischer Phosphatase (AP), Oct-4, SSEA-1, SSEA-4, TRA-1-60, TRA-1-81, NANOG, SOX2, und einem Differenzierungsmarker sind, und wobei der Differenzierungsmarker einer oder mehr aus NF-200, Brachyury, ATBF1 und MAP2 ist.

8. Verfahren nach einem der vorangehenden Ansprüche, wobei die humanen pluripotenten Stammzellen eines oder mehrere Differenzierungsgene exprimieren, und wobei die Differenzierungsgene GDF9, GATA4, HAND1 oder TUJ-1-Gene sind.

9. Verfahren nach einem der vorangehenden Ansprüche, wobei MYC in den humanen pluripotenten Stammzellen heruntergeregelt ist.

10. Verfahren nach einem der vorangehenden Ansprüche, wobei die humanen pluripotenten Stammzellen embryoide Körper bilden.

## Revendications

1. Procédé d'expansion non oncogène de cellules souches pluripotentes humaines, comprenant la coculture de cellules souches pluripotentes humaines avec des cellules souches dérivées de cordon ombilical dans un milieu, dans lequel les cellules souches dérivées de cordon ombilical sont des cellules souches mésenchymateuses humaines dérivées de cordon ombilical (HUCMSC), et le milieu comprend 2 mM de L-glutamine.

2. Procédé selon la revendication 1, dans lequel les cellules souches mésenchymateuses humaines dérivées de cordon ombilical forment une couche nourricière dans un milieu pour l'expansion des cellules souches pluripotentes humaines.

3. Procédé selon la revendication 1 ou 2, dans lequel les cellules souches mésenchymateuses humaines dérivées de cordon ombilical maintiennent les cellules souches pluripotentes humaines dans un état non différencié.

4. Procédé selon l'une quelconque des revendications 1 à 3, dans lequel les cellules souches mésenchymateuses humaines dérivées de cordon ombilical sont positives pour un ou plusieurs éléments parmi CD10, CD13, CD29, CD44, CD73, CD90, CD166 et HLA-ABC, et/ou dans lequel les cellules souches mésenchymateuses humaines dérivées de cordon ombilical sont négatives pour un ou.plusieurs éléments parmi CD1q, CD3, CD34, CD45, CD56, CD117 et HLA-DR.

5. Procédé selon l'une quelconque des revendications précédentes, dans lequel les cellules souches mésenchymateuses humaines dérivées de cordon ombilical présentent une différenciabilité ostéogénique ou adipogène.

6. Procédé selon l'une quelconque des revendications précédentes, dans lequel les cellules souches pluripotentes humaines sont des cellules souches embryonnaires humaines (hES).

7. Procédé selon l'une quelconque des revendications précédentes, dans lequel les cellules souches pluripotentes humaines sont positives pour un ou plusieurs éléments parmi phosphatase alcaline (AP), Oct-4, SSEA-1, SSEA-4, TRA-1-60, TRA-1-81, NANOG, SOX2 et un marqueur de différenciation, et dans lequel le marqueur de différenciation est un ou plusieurs éléments parmi NF-200, Brachyury, ATBF1 et MAP2.

8. Procédé selon l'une quelconque des revendications précédentes, dans lequel les cellules souches pluripotentes humaines expriment un ou plusieurs gènes de différenciation, et dans lequel les gènes de différenciation sont les gènes GDF9, GATA4, HAND1 ou TUJ-1.

9. Procédé selon l'une quelconque des revendications précédentes, dans lequel MYC est sous-exprimé dans les cellules souches pluripotentes humaines.

10. Procédé selon l'une quelconque des revendications précédentes, dans lequel les cellules souches pluripotentes humaines forment des corps embryoïdes.
